# FASCICULE DE BREVET EUROPEEN

(11) **EP 0 876 350 B1**
(45) Date de publication et mention de la délivrance du brevet: **11.07.2001**
(21) Numéro de dépôt: 96939962.5
(22) Date de dépôt: 21.11.1996
(51) Int. Cl.: C07D 231/14, A61K 31/415

(54) **DERIVES DU PYRAZOLE, PROCEDE POUR LEUR PREPARATION ET COMPOSITIONS PHARMACEUTIQUES LES CONTENANT**
PYRAZOL-DERIVATE, VERFAHREN FÜR IHRE HERSTELLUNG UND SIE ENTHALTENDE ZUSAMMENSETZUNGEN
PYRAZOLE DERIVATIVES, METHOD FOR PREPARING SAME, AND PHARMACEUTICAL COMPOSITIONS CONTAINING SAID DERIVATIVES

(30) Priorité: 23.11.1995 FR 9513956
(43) Date de publication de la demande: 11.11.1998
(73) Titulaire: SANOFI-SYNTHELABO, 75013 Paris (FR)
(72) Inventeur: BARTH, Francis, F-34070 Montpellier (FR); CONGY, Christian, F-34980 Saint-Gely-du-Fesc (FR); MARTINEZ, Serge, F-34000 Montpellier (FR); RINALDI, Murielle, F-34680 Saint-Georges-d'Orques (FR)
(74) Mandataire: Gillard, Marie-Louise
(86) Numéro de dépôt international: FR9601847
(87) Numéro de publication internationale: WO9719063

(56) Documents cités:
- EP-A- 0 477 049
- EP-A- 0 576 357
- EP-A- 0 647 629
- EP-A- 0 656 354
- EP-A- 0 658 546
- US-A- 3 449 350

## Description

La présente invention concerne de nouveaux dérivés du pyrazole et leurs sels, un procédé pour leur préparation et des compositions pharmaceutiques les contenant.

De nombreux dérivés du pyrazole ont été décrits dans la littérature ; plus particulièrement EP-A-268554 et DE-A-3910248 revendiquent des pyrazoles possédant des propriétés herbicides, EP-A-430186 et JP-A-3031840 revendiquent des composés utiles pour la photographie et EP-A-418845 revendique des pyrazoles pourvus d'activité antiinflammatoire, analgésique et antithrombotique.

Les demandes de brevet EP-A-576 357 et EP-A-658 546 décrivent des dérivés du pyrazole présentant une affinité pour les récepteurs aux cannabinoïdes. Plus particulièrement, la demande de brevet EP-A-656 354 revendique le N-pipéridino-5-(4-chlorophényl)-1-(2,4-dichlorophényl)-4-méthylpyrazole-3-carboxamide ou SR 141 716 et ses sels pharmaceutiquement acceptables qui présentent une très bonne affinité pour les récepteurs centraux aux cannabinoïdes.

On a maintenant trouvé de nouveaux dérivés du pyrazole-3-carboxamide qui possèdent une très bonne affinité pour les récepteurs centraux des cannabinoïdes et sont utiles dans les domaines thérapeutiques où le cannabis est connu pour intervenir.

Ces composés peuvent également être utilisés comme outils pharmacologiques, chez l'animal ou chez l'homme, pour la détection et le marquage des récepteurs centraux aux cannabinoïdes.

Le Δ⁹-THC est le principal constituant actif extrait de *Cannabis sativa* (Tuner, 1985 ; In Marijuana 84, Ed. Harvey, DY, IRL Press, Oxford).

Les effets des cannabinoïdes sont dûs à une intéraction avec des récepteurs spécifiques de haute affinité présents au niveau central (Devane et al., Molecular Pharmacology, 1988, 34, 605-613) et périphérique (Nye et al., The Journal of Pharmacology and Experimental Therapeutics, 1985, 234, 784-791 ; Kaminski et al., 1992, Molecular Pharmacology, 42, 736-742 ; Munro et al., Nature, 1993, 365, 61-65).

La caractérisation des récepteurs au niveau central a été rendue possible par la mise au point de ligands synthétiques des récepteurs aux cannabinoïdes tels que les agonistes WIN 55212-2 (J. Pharmacol. Exp. Ther., 1993, 264, 1352-1363) ou le CP 55,940 (J. Pharmacol. Exp. Ther., 1988, 247, 1046-1051).

Selon un de ses aspects, la présente invention concerne les composés de formule : 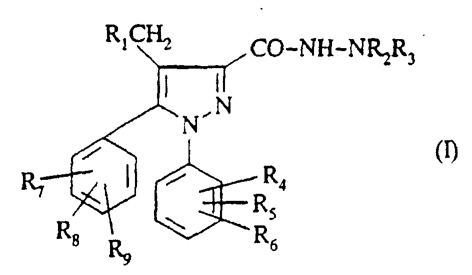 dans laquelle :
- R₁ représente un fluor, un hydroxy, un (C₁-C₅)alcoxy, un (C₁-C₅)alkylthio, un hydroxy(C₁-C₅)alcoxy, un groupe -NR₁₀R₁₁, un cyano, un (C₁-C₅)alkylsulfonyle, un (C₁-C₅)alkylsulfinyle ;
- R₂ et R₃ représentent un (C₁-C₄)alkyle ou ensemble avec l'atome d'azote auquel ils sont liés constituent un radical hétérocyclique, saturé ou insaturé, de 5 à 10 chaînons, non substitué ou substitué une ou plusieurs fois par un (C₁-C₃)alkyle ou par un (C₁-C₃)alcoxy;
- R₄, R₅, R₆, R₇, R₈, R₉ représentent chacun indépendamment l'hydrogène, un halogène, un trifluorométhyle, et lorsque R₁ représente un fluor, R₄, R₅, R₆, R₇, R₈ et/ou R₉ peuvent également représenter un fluorométhyle ; et à la condition que l'un au moins des substituants R₄ ou R₇ soit différent de l'hydrogène ;
- R₁₀ et R₁₁ représentent chacun indépendamment l'hydrogène ou un (C₁-C₅)alkyle ou R₁₀ et R₁₁ ensemble avec l'atome d'azote auquel ils sont liés constituent un radical hétérocyclique choisi parmi pyrrolidin-1-yle, pipéridin-1-yle, morpholin-4-yle, pipérazin-1-yle non substitué ou substitué par un (C₁-C₄)alkyle ;
ainsi que leurs sels et leurs solvates.

Par (C₁-C₃)alkyle, (C₁-C₅)alkyle, (C₁-C₅)alcoxy, (C₁-C₅)alkylthio, on entend des radicaux alkyles ou respectivement des radicaux alcoxy ou alkylthio en C₁-C₃ ou C₁-C₅ droits ou ramifiés.

Par halogène, on entend chlore, fluor, brome ou iode, préférentiellement le chlore.

Par radical hétérocyclique saturé ou insaturé de 5 ou 10 chaînons, on entend un radical hétérocyclique non aromatique mono, di ou tricyclique, condensé ou ponté. Ces radicaux comprennent en particulier les radicaux suivants : pyrrolidin-1-yle, pipéridin-1-yle, hexahydroazépin-1-yle, 2-azabicyclo[2.2.2]oct-5-èn-2-yle, 2-azaadamant-2-yle, 1,2,3,6-tétrahydropyridin-1-yle, 2-azabicyclo[2.2.1]heptan-2-yle, 2-azabicyclo[2.2.2]octan-2-yle.

Les sels du composé de formule (I) comprennent les sels d'addition d'acides pharmaceutiquement acceptables tels que le chlorhydrate, le bromhydrate, le sulfate, l'hydrogénosulfate, le dihydrogénophosphate, le méthanesulfonate, le méthylsulfate, le maléate, l'oxalate, le fumarate, le 2-naphtalènesulfonate, le glyconate, le gluconate, le citrate, l'iséthionate, le paratoluènesulfonate.

Avantageusement, la présente invention a pour objet les composés de formule : 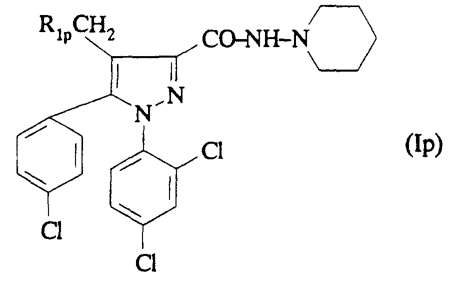 dans laquelle R₁ₚ représente un fluor, un méthoxy ou un méthylthio, ainsi que leurs sels et leurs solvates.

Selon un autre de ses aspects, la présente invention concerne un procédé pour la préparation des composés de formule (I) ci-dessus, de leurs sels et de leurs solvates ; ce procédé appelé Procédé 1 est caractérisé en ce que :
a) on traite un ester bromé de formule : 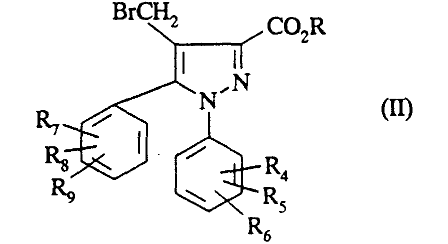 dans laquelle R₄, R₅, R₆, R₇, R₈, R₉ sont tels que définis ci-dessus et R représente un (C₁-C₄)alkyle, par un composé de formule : R'₁ A (III) dans laquelle R'₁ représente R₁ tel que défini pour (I) ou un précurseur de R₁ et A représente un hydrogène ou un cation ;
b1) dans l'ester ainsi obtenu, on transforme éventuellement R'₁ en R₁ ;
b2) on saponifie l'ester ainsi obtenu à l'étape a) ou à l'étape b1), de formule : 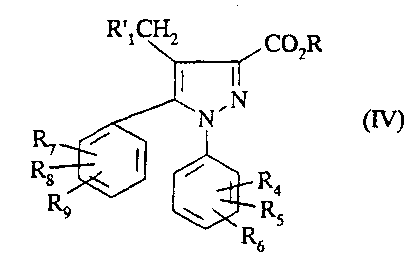
c) on traite l'acide ainsi obtenu de formule : 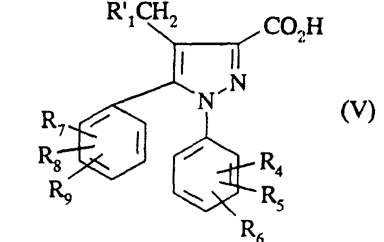 ou un dérivé fonctionnel de cet acide, avec une hydrazine de formule : H₂N-NR₂R₃ (VI) dans laquelle R₂ et R₃ sont tels que définis pour (I) ;
d1) dans le composé ainsi obtenu, on transforme éventuellement R'₁ en R₁ ;
d2) éventuellement, on transforme le composé obtenu à l'étape c) ou l'étape d1) de formule : 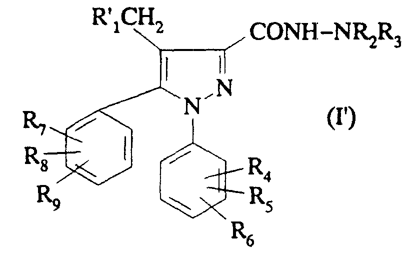 en l'un de ses sels ou de leurs solvates.

Par précurseur de R₁, on entend un groupe qui peut être transformé en R₁ dans une étape ultérieure. Cette transformation peut être réalisée sur l'ester de formule (IV) obtenu à l'étape a) ou sur le composé de formule (I') obtenu à l'étape c).

Ainsi selon une variante du procédé, appelée procédé 2, on peut préparer un composé de formule (I) dans laquelle R₁ = R₁ₐ et représente un groupe choisi parmi un fluor, un (C₁-C₅)alcoxy, un (C₁-C₅)alkylthio, un hydroxy(C₁-C5)alcoxy, un cyano, un groupe NR₁₀R₁₁ dans lequel R₁₀ et R₁₁ sont tels que définis ci-dessus, à partir d'un composé de formule (I') dans laquelle R'₁ = OH.

Les esters bromés de formule (II) sont décrits dans la demande de brevet européenne EP-A-658 546. Ils sont préparés par action de la N-bromosuccinimide dans un solvant comme le tétrachlorure de carbone, en présence de péroxyde de benzovle sur un composé de formule :

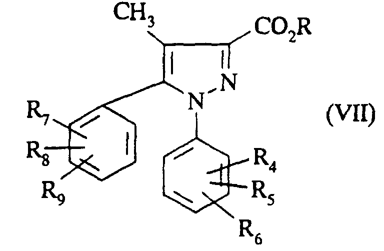

La préparation des composés de formule (VII) est également décrite dans la demande de brevet EP-A-658 546.

Les hydrazines H₂N-NR₂R₃ sont connues ou préparées par des méthodes connues.
Par exemple, on peut les obtenir selon le procédé décrit dans Chem. Ber., 1986, 119, 1413-1423, qui consiste en la réduction d'un dérivé nitroso de formule : ON-NR₂R₃ (VIII) dans laquelle R₂ et R₃ sont tels que définis ci-dessus pour (I), par un hydrure tel que l'hydrure d'aluminium et de lithium. Le dérivé nitroso (VIII) s'obtient par réaction d'un composé de formule : HNR₂R₃ (IX) dans laquelle R₂ et R₃ sont tels que définis ci-dessus pour (I), avec le nitrite de sodium en solution aqueuse en présence d'un acide tel que l'acide acétique.

La 1-aminopipéridine est commerciale.

Le 2-amino-2-azabicyclo[2.2.2]oct-5-ène se prépare selon Chem. Ber., 1986, 119, 1413-1423.

Le 2-amino-2-azaadamantane se prépare à partir du 2-azaadamantane, via le dérivé nitroso. Le 2-azaadamantane se prépare selon J. Org. Chem., 1981, 46, 4953.

Dans la définition de A, par cation, on entend un cation de métal alcalin ou alcalino-terreux, ou un groupe ammonium quaternaire tel que le tétraéthylammonium.

Comme dérivé fonctionnel de l'acide (V) on peut utiliser le chlorure d'acide, l'anhydride, un anhydride mixte, un ester alkylique en C₁-C₄ dans lequel l'alkyle est droit ou ramifié, un ester activé, par exemple l'ester de *p*-nitrophényle, ou l'acide libre opportunément activé, par exemple, avec le N,N-dicyclohexylcarbodiimide, l'hexafluorophosphate de benzotriazol-N-oxotris(diméthylamino)phosphonium (BOP) ou le tétrachlorure de silicium (Synth. Commun., 1986, 16, 1261).

Ainsi dans le procédé selon l'invention, à l'étape c) on peut faire réagir le chlorure de l'acide pyrazole-3-carboxylique, obtenu par réaction du chlorure de thionyle sur l'acide de formule (V), avec une hydrazine H₂N--NR₂R₃ (VI), dans un solvant tel que le dichlorométhane, sous une atmosphère inerte, à une température comprise entre 0° C et la température ambiante, en présence d'une base telle que la triéthylamine.

Une variante au mode opératoire de l'étape c) consiste à préparer l'anhydride mixte de l'acide de formule (V) par réaction du chloroformiate d'éthyle avec l'acide de formule (V), en présence d'une base telle que la triéthylamine, et à le faire réagir avec l'hydrazine, dans un solvant tel que le dichlorométhane, sous une atmosphère inerte, à la température ambiante, en présence d'une base telle que la triéthylamine.

Pour préparer un composé de formule (I) dans lequel R₁ = OH, on utilise comme réactif R'₁A (III) un hydroxyde de métal alcalin ou alcalino-terreux tel que la soude ou la potasse, on obtient alors directement l'acide de formule (V).

Ainsi le procédé 2 selon l'invention est caractérisé en ce que :
e) on traite un ester bromé de formule : 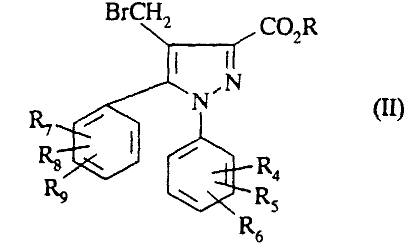 dans laquelle R₄, R₅, R₆, R₇, R₈, R₉ et R sont tels que définis ci-dessus par un hydroxyde de métal alcalin ou alcalino-terreux ;
f) on traite l'acide ainsi obtenu de formule : 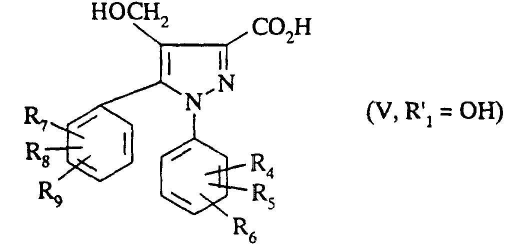 ou un dérivé fonctionnel de cet acide, avec une hydrazine de formule H₂N-NR₂R₃ (VI) dans laquelle R₂ et R₃ sont tels que définis pour (I) ;
g) on traite le composé ainsi obtenu de formule : 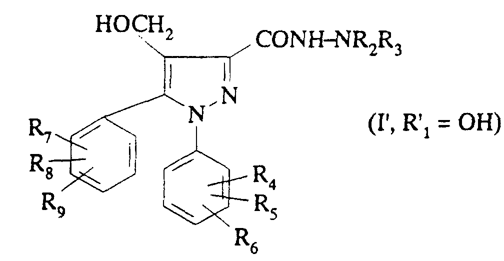 par un composé de formule Hal-B dans laquelle Hal représente un halogène, de préférence le chlore et B représente un radical mésyle, tosyle ou trifluorométhanesulfonyle ;
h) on traite le composé ainsi obtenu de formule: 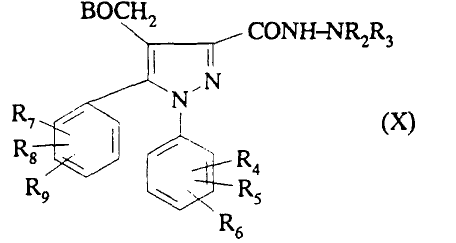 par un composé de formule R₁ₐ A (XI) dans laquelle R₁ₐ est tel que défini ci-dessus et A représente un hydrogène ou un cation ;
i) et, éventuellement, on transforme le composé ainsi obtenu de formule : 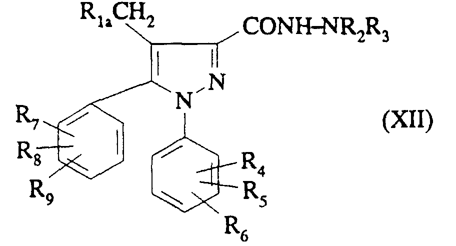 en l'un de ses sels ou de leurs solvates.

Pour préparer un composé de formule (I) dans laquelle R₁ est un (C₁-C₅)alcoxy, on utilise comme réactif de formule (III) ou (XI), un alcool en (C₁-C₅) en présence d'une base non nucléophile telle qu'un hydrure métallique comme l'hydrure de sodium ou de potassium. Selon les valeurs de R₁ et R on obtient à l'étape a) du procédé 1 un mélange d'esters qui est saponifié à l'étape b2) pour donner l'acide de formule (V).

Pour préparer un composé de formule (I) dans laquelle R₁ est un (C₁-C₅)alkylthio, on utilise comme réactif de formule (III) ou (XI) un thioalcool en (C₁-C₅)en présence d'une base non nucléophile telle qu'un hydrure métallique comme l'hydrure de sodium ou de potassium.

Le cas échéant, on peut transformer l'ester de formule (IV) obtenu à l'étape a) ou respectivement le composé de formule (XII) obtenu à l'étape h) dans lequel R'₁ ou respectivement R₁ₐ est un (C₁-C₅)alkylthio par action d'un agent oxydant tel que l'eau oxygénée ou l'acide *meta*chloroperbenzoïque pour obtenir un composé de formule (IV) ou respectivement de formule (I) dans lequel R₁ représente un (C₁-C₅)alkylsulfonyle ou un (C₁-C5)alkylsulfinyle.

Pour préparer un composé de formule (I) dans lequel R₁ est un cyano, on peut utiliser comme réactif de formule (III) ou (XI), un cyanure métallique tel que le cyanure de sodium ou un cyanure d'ammonium quaternaire, par exemple le cyanure de tétraéthylammonium ; dans ce dernier cas la réaction de substitution nucléophile de l'étape a) ou de l'étape h) est réalisée en présence d'un catalyseur de transfert de phase.

Pour préparer un composé de formule (I) dans laquelle R₁ est le fluor, on peut utiliser comme réactif de formule (III) ou (XI) un agent de fluorination ; comme agent de fluorination, on peut citer un fluorure d'ammonium quaternaire tel que le fluorure de tétrabutylammonium ; un fluorure métallique, par exemple le fluorure de potassium utilisé en présence d'une base faible comme le carbonate de potassium et d'un agent complexant comme le Kryptofix® ; un complexe d'acide fluorhydrique, par exemple un complexe (HF)ₙ-amine tertiaire avec n = 1, 2, 3, tel que (HF)₃-N(C₂H₅)₃. Préférentiellement, on prépare un composé de formule (I) dans laquelle R₁ = F à partir d'un composé de formule (I) dans laquelle R₁ = OH par fluorination : on peut soit réaliser une fluorination directe, par exemple avec le trifluorure de diéthylaminosulfure, soit, selon le procédé 2, préparer de façon intermédiaire un composé de formule (X) puis effectuer la fluorination par un agent tel qu'un agent de fluorination décrit ci-dessus.

Plus particulièrement, on préfère préparer un composé de formule (I) dans laquelle R₁ = F par le procédé 1 à partir d'un ester de formule (II) et en utilisant comme composé de formule (III) le fluorure de potassium (R'₁A = KF) en présence d'un agent complexant comme le Kryptofix®.

De façon particulière, on prépare un composé de formule (I) dans laquelle R₁ représente un fluor et R₄, R₅, R₆, R₇, R₈ et/ou R₉ représente un fluorométhyle en utilisant comme produit de départ un composé de formule (II) dans laquelle R₄, R₅, R₆, R₇, R₈ et/ou R₉ représente un bromométhyle. La fluorination effectuée selon l'un des procédés décrits ci-dessus permet de transformer R₄, R₅, R₆, R₇, R₈ et/ou R₉ en fluorométhyle. Le composé de départ (II) est préparé par action de la N-bromosuccinimide sur un composé de formule (VII) dans laquelle R₄, R₅, R₆, R₇, R₈ et/ou R₉ représente un méthyle.

Pour préparer un composé de formule (I) dans laquelle R₁ est un groupe -NR₁₀R₁₁ on peut faire réagir à l'étape a) une amine de formule : HNR₁₀R₁₁ (III). Préférentiellement, on prépare un composé de formule (I) dans laquelle R₁ = NR₁₀R₁₁ selon le procédé 2 à partir d'un composé de formule (I') dans laquelle R'₁ = OH par transformation intermédiaire de l'alcool en mésyloxy, en tosyloxy ou en trifluorométhanesulfonyloxy puis action d'une amine HNR₁₀R₁₁, en présence d'une base non nucléophile telle qu'un hydrure métallique, comme l'hydrure de sodium.

Le composé de formule (I) obtenu par le procédé selon l'invention est isolé, sous forme d'une base libre ou de sel ou de solvate, selon les techniques conventionnelles.

Le composé de formule (I) peut être isolé sous forme d'un de ses sels, par exemple le chlorhydrate ou l'oxalate ; dans ce cas, la base libre peut être préparée par neutralisation dudit sel avec une base minérale ou organique, telle que l'hydroxyde de sodium ou d'ammonium, la triéthylamine ou un carbonate ou bicarbonate alcalin tel que le carbonate ou le bicarbonate de sodium ou de potassium, et transformée dans un autre sel comme le méthanesulfonate, le fumarate ou le 2-naphtalènesulfonate.

Lorsque le composé (I) est obtenu sous forme de base libre, la salification est effectuée par traitement avec l'acide choisi dans un solvant organique. Par traitement de la base libre, dissoute par exemple dans un éther tel que l'éther diéthylique ou dans l'acétone, avec une solution de l'acide dans le même solvant, on obtient le sel correspondant qui est isolé selon les techniques classiques.

Les composés de formule (I) possèdent une très bonne affinité *in vitro* pour les récepteurs aux cannabinoïdes centraux, dans les conditions expérimentales décrites par Devane et al., Molecular Pharmacology, 1988, 34, 605-613.

Plus particulièrement, les composés de la présente invention, tels quels ou sous forme d'un de leurs sels pharmaceutiquement acceptables, sont des antagonistes puissants et sélectifs des récepteurs aux cannabinoïdes centraux, ayant un Ki compris entre 1 et 100 nM. Ils sont entre 100 et 1000 fois plus actifs sur les récepteurs centraux que sur les récepteurs périphériques, sont actifs par voie orale et passent la barrière hématoencéphalique.

La bonne pénétration des composés de la présente invention dans le système nerveux central a été mise en évidence par les expériences de liaison ex-vivo au [³H]-CP55940 dans les conditions décrites par M. Rinaldi-Carmona et al., Life Sciences, 1995, 56, 1941-1947.

D'autre part leur nature antagoniste a été démontrée par les résultats dans les modèles de l'inhibition de l'adenylate cyclase et de la contraction du vas deferens de souris comme décrit dans M. Rinaldi-Carmona et al., FEBS Letters, 1994, 350, 240-244.

Grâce à leurs remarquables propriétés, notamment à leur grande affinité, à leur sélectivité pour le récepteur central et à leur capacité à pénétrer la barrière hématoencéphalique, les composés (I), tels quels ou éventuellement sous forme de sels pharmaceutiquement acceptables ou de solvates, peuvent être utilisés comme principes actifs de médicaments destinés à combattre les maladies du système nerveux central des mammifères.

La toxicité des composés (I) est compatible avec leur utilisation en tant que médicaments psychotropes, notamment pour le traitement des troubles thymiques, des troubles anxieux, des troubles de l'humeur, du vomissement, des troubles mnésiques, des troubles cognitifs, des neuropathies, de la migraine, du stress, des maladies d'origine psychosomatique, de l'épilepsie, des dyskinésies ou de la maladie de Parkinson.

Les composés (I) selon l'invention peuvent également être utilisés en tant que médicament pour le traitement des troubles de l'appétit notamment en tant qu'anorexigène, pour le traitement de la schizophrénie, des troubles délirants, des troubles psychotiques en général, ainsi que des troubles liés à l'utilisation de substances psychotiques. De plus, les composés (I) selon l'invention peuvent être utilisés en tant que médicament pour la chimiothérapie anticancéreuse.

De plus, les composés (I) selon l'invention, tels quels ou sous forme radiomarquée peuvent être utilisés comme outils pharmacologiques chez l'homme ou chez l'animal, pour la détection et le marquage des récepteurs centraux aux cannabinoïdes. En particulier les composés de formule (I) dans laquelle R₁ est un atome de fluor radioactif tel que ¹⁸F peuvent être utilisés dans des études de tomographie par émission de positron afin de visualiser in vivo la localisation et la densité des récepteurs aux cannabinoïdes centraux et d'étudier la pharmacocinétique et la biodistribution d'agonistes ou d'antagonistes des récepteurs aux cannabinoïdes centraux.

D'autre part les composés de formule (I) contenant un groupe OH tels que ceux comprenant un groupe R₁ = OH ou R₁ = hydroxy(C₁-C₅)alcoxy peuvent servir d'intermédiaires pour la préparation de ligands irréversibles comprenant des groupes photoactivables ou des groupes électrophiles tels que par exemple un azido, un isothiocyanato, un haloacétamido, un accepteur de Michaël ou un ester d'aldol. Ces ligands irréversibles peuvent être utilisés pour isoler, purifier et caractériser les récepteurs aux cannabinoïdes, et identifier leur site actif.

Les composés de formule (I) contenant un groupe OH tels que ceux comprenant un groupe R₁ = OH ou R₁ = hydroxy(C₁-C₅)alcoxy peuvent également servir d'intermédiaires pour la préparation de ligands des récepteurs aux cannabinoïdes centraux contenant un groupement détectable par des méthodes immunochimiques, tel que par exemple le groupe biotinyl. Ces ligands peuvent être utilisés pour la détection, la caractérisation et la purification des récepteurs aux cannabinoïdes centraux.

Les composés selon l'invention sont généralement administrés en unité de dosage.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables ou un de leurs solvates.

Les composés de formule (I) ci-dessus et leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration orale, sublinguale, sous-cutanée, intramusculaire, intraveineuse, topique, transdermique, locale ou rectale, le principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration topique, les formes d'administration sous-cutanée, intramusculaire, intraveineuse, intranasale ou intraoculaire et les formes d'administration rectale.

Dans les compositions pharmaceutiques de la présente invention, le principe actif est généralement formulé en unités de dosage contenant de 0,5 à 1000 mg, avantageusement de 1 à 500 mg, de préférence de 2 à 200 mg dudit principe actif par unité de dosage pour les administrations quotidiennes.

Lorsque l'on prépare une composition solide sous forme de comprimé, on peut ajouter au principe actif, micronisé ou non, un agent mouillant tel que le laurylsulfate de sodium et on mélange le tout avec un véhicule pharmaceutique tel que la silice, l'amidon, le lactose, le stéarate de magnésium, le talc ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif en mélange avec des agents de dispersion, des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le polyéthylèneglycol.

Ainsi, pour préparer une solution aqueuse injectable par voie intraveineuse on peut utiliser un cosolvant : un alcool tel que l'éthanol, un glycol tel que le polyéthylèneglycol ou le propylèneglycol et un tensioactif hydrophile tel que le Tween® 80. Pour préparer une solution huileuse injectable par voie intramusculaire, on peut solubiliser le principe actif par un triglycéride ou un ester de glycérol.

Pour l'administration locale, on peut utiliser des gels, des pommades ou des crèmes.

Pour l'administration transdermique, on peut utiliser des patches sous forme multilaminé ou à réservoirs dans lesquels le principe actif est en solution alcoolique.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple α-, β- ou γ- cyclodextrine, 2-hydroxypropyl-β-cyclodextrine ou méthyl-β-cyclodextrine.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Les exemples suivants illustrent l'invention sans toutefois la limiter.

Les points de fusion ou de décomposition des produits, F, sont mesurés en tube capillaire avec un appareil de Tottoli.

Les spectres de RMN sont enregistrés à 200 MHz dans le DMSO.

Dans les préparations et dans les exemples, les abréviations suivantes sont utilisées:
THF: tétrahydrofurane
CCl₄ : tétrachlorure de carbone
Ether : éther diéthylique
(iPr)₂O : Ether iso : éther diisopropylique
EtOH : éthanol
AcOEt : acétate d'éthyle
MeOH : méthanol
DCM: dichlorométhane
NaOH : soude
NaHCO₃ : carbonate acide de sodium
NH₄Cl : chlorure d'ammonium
NaH : hydrure de sodium
MgSO₄ : sulfate de magnésium
KOH : potasse
Kryptofix® : 4, 7, 13, 16, 21, 24-hexaoxa-1, 10-diazabicyclo[8.8.8]-hexacosane
AcOH : acide acétique
HCl : acide chlorhydrique
H₂SO₄ : acide sulfurique
NaCl : chlorure de sodium
BOP : hexafluorophosphate de benzotriazol N-oxotris(diméthylamino) phosphonium
TA : température ambiante
F : point de fusion
TFA : acide trifluoroacétique

Pour l'interprétation des spectres RMN on utilise les abréviations suivantes :
s : singulet
s.e. : singulet élargi
d : doublet
d. de d. : doublet de doublet
t : triplet
q : quadruplet
m : multiplet ou massif.

### PREPARATION 1 :

### Ester méthylique de l'acide 5-(4-chlorophényl)-1-(2,4-dichlorophényl)-4-bromométhylpyrazole-3-carboxylique.

A) Ester méthylique de l'acide 5-(4-chlorophényl)-1-(2,4-dichlorophényl)-4-méthylpyrazole-3-carboxylique.
   19,54 g d'acide 5-(4-chlorophényl)-1-(2,4-dichlorophényl)-4-méthylpyrazole-3-carboxylique sont dissous dans 350 ml de MeOH, on ajoute 0,97 g d'acide *para*-toluènesulfonique puis on chauffe à reflux pendant 18 heures. Après évaporation du solvant, le résidu est repris dans AcOEt, lavé par une solution saturée de NaHCO₃, puis par une solution saturée de NaCl. On sèche sur MgSO₄, filtre l'insoluble puis concentre à sec pour obtenir 18,53 g du composé attendu, F = 133°C.
B) Ester méthylique de l'acide 5-(4-chlorophényl)-1-(2,4-dichlorophényl)-4-bromométhylpyrazole-3-carboxylique.
   A une solution de 9 g du composé de l'étape précédente dans 110 ml de CCl₄, on ajoute 4,05 g de N-bromosuccinimide et 20 mg de péroxyde de benzoyle et on chauffe à reflux pendant 19 heures. La solution est filtrée puis le filtrat est lavé à l'eau, avec une solution saturée de NaCl, puis séché sur MgSO₄. Après évaporation du solvant, la mousse obtenue est reprise à TA par (iPr)₂O et séchée sous vide. Le solide blanc obtenu est recristallisé dans (iPr)₂O pour donner 5,96 g du composé attendu, F = 145°C.

### PREPARATION 2

### Acide 5-(4-chlorophényl)-1-(2,4-dichlorophényl)-4-hydroxyméthylpyrazole-3-carboxylique.

On place 2 g du composé de la PREPARATION 1 dans 30 ml de THF, on ajoute 30 ml de NaOH à 5 % puis on chauffe à reflux pendant 17 heures. Après refroidissement, on verse le milieu réactionnel dans 200 ml d'HCl à 5 %, à O'C, extrait au DCM, lave par une solution saturée de NaCl puis sèche sur MgSO₄. On obtient 1,79 g du produit attendu (solide amorphe).

### PREPARATION 3

### Acide 5-(4-chlorophényl)-1-(2,4-dichlorophényl)-4-((2-hydroxyéthoxy)méthyl)pyrazole-3-carboxylique.

A) 5-(4-Chlorophényl)-1-(2,4-dichlorophényl)-4-((2-hydroxyéthoxy)méthyl) pyrazole-3-carboxylate de 2-hydroxyéthyle.
   On place 2 g du composé de la PREPARATION 1 dans 80 ml de THF et l'on ajoute sous azote, à 0°C, 1,2 ml d'éthylèneglycol et 201 mg de NaH en 2 fois. On laisse revenir à TA puis on chauffe à reflux pendant 20 heures. Après évaporation du solvant, on reprend par une solution saturée de NH₄Cl, extrait par AcOEt, lave à l'eau et sèche sur MgSO₄. Le résidu est chromatographié sur silice en éluant par MeOH/DCM (1/99 à 2/98 ; v/v). On obtient 640 mg du produit attendu.
B) Acide 5-(4-chlorophényl)-1-(2,4-dichlorophényl)-4-((2-hydroxyéthoxy)méthyl) pyrazole-3-carboxylique.
   On place 670 mg du composé obtenu à l'étape précédente dans 20 ml de MeOH puis on ajoute 0,19 g de potasse dans 18 ml d'eau et on porte à reflux pendant 5 heures. Après retour du milieu réactionnel à TA, on ajoute 100 ml d'HCl à 5 %. Le précipité formé est filtré, lavé à l'eau et séché sous vide pour donner 420 mg du composé attendu, F = 182°C.

### PREPARATION 4

### Acide 5-(4-chlorophényl)-1-(2,4-dichlorophényl)-4-(méthylthiométhyl)pyrazole-3-carboxylique.

A) Ester méthylique de l'acide 5-(4-chlorophényl)-1-(2,4-dichlorophényl)-4-(méthylthiométhyl)pyrazole-3-carboxylique.
   On place 1,09 g du composé de la PREPARATION 1 dans 15 ml de THF et l'on ajoute 0,19 g de méthylthiolate de sodium. Après 6 jours sous agitation à TA, on dilue par 50 ml d'eau puis on extrait par AcOEt et lave par une solution saturée de NaCl. On obtient 0,90 g du composé attendu (solide amorphe).
B) Acide 5-(4-chlorophényl)-1-(2,4-dichlorophényl)-4-(méthylthiométhyl)pyrazole-3-carboxylique.
   Le composé de l'étape précédente (0,90 g) est placé dans 15 ml de MeOH, on ajoute 0,29 g de potasse dans 10 ml d'eau et on chauffe à reflux pendant 3 heures. Le milieu réactionnel est versé sur 100 ml d'eau glacée et acidifié à pH = 2 par addition d'HCl à 5 %. Le solide blanc obtenu est filtré, lavé à l'eau et séché sous vide. On obtient 0,78 g du produit attendu (solide amorphe).

### PREPARATION 4 bis.

### Chlorure de l'acide 5-(4-chlorophényl)-1-(2,4-dichlorophényl)-4-(méthylthio méthyl)pyrazole-3-carboxylique.

On place 0,78 g de l'acide de la PREPARATION 4 dans 15 ml de toluène et l'on ajoute 0,49 ml de chlorure de thionyle puis on chauffe à reflux pendant 3 heures. On évapore le solvant sous vide, reprend le résidu par 30 ml de toluène et évapore à nouveau sous vide (2 fois). On obtient 1 g du produit attendu.

### PREPARATION 5

### Acide 5-(4-chlorophényl)-1-(2,4-dichlorophényl)-4-méthoxyméthylpyrazole-3-carboxylique.

On prépare une solution contenant 1 g du composé de la PREPARATION 1 dans 30 ml de MeOH et 0,14 g de méthylate de sodium et on chauffe 18 heures à reflux. On ajoute 0,24 g de potasse dans 5 ml d'eau et chauffe encore 2 heures à reflux. On concentre sous vide, dilue dans 20 ml d'eau et acidifie à pH = 2 par addition d'HCl à 10 %. Le solide blanc formé est filtré et lavé à l'eau pour donner 0,82 g du produit attendu, F = 95°C.

### PREPARATION 5 bis

### Chlorure de l'acide 5-(4-chlorophényl)-1-(2,4-dichlorophényl)-4-méthoxyméthylpyrazole-3-carboxylique.

L'acide de la PREPARATION 5 (0,82 g) est placé dans 30 ml de toluène, on ajoute 0,54 ml de chlorure de thionyle puis on chauffe à reflux pendant 2 heures. On évapore à sec, reprend dans 20 ml de toluène et évapore à sec à nouveau (2 fois). La mousse blanche obtenue est utilisée directement dans une étape ultérieure pour préparer un composé selon l'invention.

### PREPARATION 6

### Ester tert-butylique de l'acide 5-(4-chlorophényl)-1-(2,4-dichlorophényl)-4-bromométhylpyrazole-3-carboxylique.

A) Ester *tert*-butylique de l'acide 5-(4-chlorophényl)-1-(2,4-dichlorophényl)-4-méthylpyrazole-3-carboxylique.
   On introduit dans un autoclave de 250 ml une solution de 5 g d'acide 5-(4-chlorophényl)-1-(2,4-dichlorophényl)-4-méthylpyrazole-3-carboxylique dans 35 ml de dioxane puis refroidit à -10°C. On ajoute ensuite 2 ml d'H₂SO₄ concentré et 70 ml de 2-méthylpropène refroidi à -10°C, ferme l'autoclave, laisse revenir à TA puis chauffe à 40°C pendant 18 heures sous forte agitation. Après refroidissement à TA, on concentre sous vide le mélange réactionnel, extrait le résidu au DCM, lave la phase organique à l'eau, par une solution à 5 % de Na₂CO₃, par une solution saturée de NaCl, sèche sur MgSO₄ et évapore sous vide le solvant. On chromatographie le résidu sur silice en éluant par le mélange toluène/AcOEt de (90/10 ; v/v) à (80/20 ; v/v). On obtient 3,15 g du produit attendu, F = 112°C.
B) Ester *tert*-butylique de l'acide 5-(4-chlorophényl)-1-(2,4-dichlorophényl)-4- bromométhylpyrazole-3-carboxylique.
   A une solution de 4,28 g du composé obtenu à l'étape précédente dans 86 ml de CCl₄, on ajoute 1,94 g de N-bromosuccinimide et 10 mg de péroxyde de benzoyle, puis chauffe à reflux pendant 16 heures. Après refroidissement à TA, on filtre l'insoluble et concentre sous vide le filtrat. On obtient 2,48 g du produit attendu, F = 139°C.

### PREPARATION 7

### Acide 5-(4-chlorophényl)-1-(2,4-dichlorophényl)-4-cyanométhylpyrazole-3-carboxylique.

A) Ester *tert*-butylique de l'acide 5-(4-chlorophényl)-1-(2,4-dichlorophényl)-4-cyanométhylpyrazole-3-carboxylique.
   A une solution de 0,6 g du composé obtenu à la PREPARATION 6 dans 20 ml de DCM on ajoute 0,229 g de cyanure de tétraéthylammonium et chauffe à reflux pendant 5 heures. On lave le mélange réactionnel à l'eau, par une solution saturée de Na₂CO₃, par une solution saturée de NaCl, sèche la phase organique sur MgSO₄ et évapore sous vide le solvant. On chromatographie le résidu sur silice en éluant par le mélange AcOEt/toluène (5/95 ; v/v). On obtient 0,55 g du produit attendu, F = 168°C.
B) Acide 5-(4-chlorophényl)-1-(2,4-dichlorophényl)-4-cyanométhylpyrazole-3-carboxylique.
   On laisse 30 minutes sous agitation à TA un mélange de 0,55 g du composé obtenu à l'étape précédente et 3 ml de TFA. On concentre sous vide, extrait le résidu au DCM, lave la phase organique par une solution saturée de NaCl, sèche sur MgSO₄ et évapore sous vide le solvant. On obtient 0,341 g du produit attendu.

### PREPARATION 7 bis

### Chlorure de l'acide 5-(4-chlorophényl)-1-(2,4-dichlorophényl)-4-cyanométhyl pyrazole-3-carboxylique.

A un mélange de 0,91 g du composé obtenu à la PREPARATION 7 et 20 ml de toluène, on ajoute goutte à goutte 0,91 ml de chlorure de thionyle puis chauffe à reflux pendant 1 heure. On concentre sous vide, reprend le résidu au toluène et évapore sous vide le solvant. On obtient 1,1 g du produit attendu.

### PREPARATION 8

### Ester éthylique de l'acide 5-(4-chlorophényl)-1-(2,4-dichlorophényl)-4-bromométhylpyrazole-3-carboxylique.

A une solution de 10,73 g d'ester éthylique de l'acide 5-(4-chlorophényl)-1-(2,4-dichlorophényl)-4-méthylpyrazole-3-carboxylique dans 200 ml de CCl₄, on ajoute 4,8 g de N-bromosuccinimide et 20 mg de péroxyde de benzoyle puis chauffe à reflux pendant 16 heures. On filtre l'insoluble et concentre sous vide le filtrat. On extrait le résidu au DCM, lave la phase organique à l'eau, sèche sur MgSO₄ et évapore sous vide le solvant. On obtient 14,13 g du produit attendu.

### PREPARATION 9

### Acide 5-(4-chlorophényl)-1-(2,4-dichlorophényl)-4-(méthylsulfonylméthyl)pyrazole-3-carboxylique.

A) Ester éthylique de l'acide 5-(4-chlorophényl)-1-(2,4-dichlorophényl)-4-(méthylthiométhyl)pyrazole-3-carboxylique.
   On chauffe à reflux pendant 5 jours une solution de 3 g du composé obtenu à la PREPARATION 8 et 0,68 g de méthylthiolate de sodium dans 40 ml de THF. On hydrolyse par ajout de 50 ml d'eau, concentre sous vide le THF, extrait la phase aqueuse restante au DCM, lave la phase organique par une solution saturée de NaCl, sèche sur MgSO₄ et évapore sous vide le solvant. On obtient 2,51 g du produit attendu.
B) Ester éthylique de l'acide 5-(4-chlorophényl)-1-(2,4-dichlorophényl)-4-(méthylsulfonylméthyl)pyrazole-3-carboxylique.
   A une solution de 2,51 g du composé obtenu à l'étape précédente, dans 25 ml de DCM, on ajoute goutte à goutte une solution de 1,96 g d'acide m-chloroperbenzoïque à 50 % dans 25 ml de DCM puis chauffe à reflux pendant 16 heures. On extrait le mélange réactionnel au DCM, lave la phase organique à l'eau, par une solution à 5 % de Na₂CO₃, par une solution saturée de NaCl, sèche sur Na₂SO₄ et évapore sous vide le solvant. On chromatographie le résidu sur silice en éluant par le mélange toluène/AcOEt (80/20 ; v/v). On obtient 0,68 g du produit attendu.
C) Acide 5-(4-chlorophényl)-1-(2,4-dichlorophényl)-4-(méthylsulfonylméthyl)pyrazole-3-carboxylique.
   A une solution de 0,65 g du composé obtenu à l'étape précédente dans 20 ml de MeOH on ajoute une solution de 0,25 g de KOH dans 20 ml d'eau puis chauffe à reflux pendant 1 heure. On verse le mélange réactionnel sur 50 ml d'une solution à 10 % d'HCl refroidie à 0°C, essore le précipité formé, le lave à l'eau et le sèche sous vide. On obtient 0,68 g du produit attendu.

### PREPARATION 9 bis

### Chlorure de l'acide 5-(4-chlorophényl)-1-(2,4-dichlorophényl)-4-(méthylsulfonylméthyl)pyrazole-3-carboxylique.

A une solution de 0,53 g du composé obtenu à la PREPARATION 9 dans 3 ml de toluène, on ajoute 0,5 g de chlorure de thionyle puis chauffe à reflux pendant 1 heure. On concentre sous vide, reprend le résidu dans 5 ml de toluène et évapore sous vide le solvant. On obtient 0,53 g du produit attendu.

### PREPARATION 10

### Acide 5-(4-chlorophényl)-1-(2,4-dichlorophényl)-4-fluorométhylpyrazole-3-carboxylique.

A) Ester méthylique de l'acide 5-(4-chlorophényl)-1-(2,4-dichlorophényl)-4-fluorométhylpyrazole-3-carboxylique.
   A une solution de 0,65 g du composé obtenu à la PREPARATION 1 dans 50 ml d'acétonitrile sec on ajoute successivement 0,51 g de Kryptofix® et 0,79 g de fluorure de potassium sec puis chauffe à reflux pendant 1 heure. On concentre sous vide, reprend le résidu par 30 ml d'eau, extrait par 30 ml d'AcOEt, lave la phase organique par une solution saturée de NaCl, sèche sur MgSO₄ et évapore sous vide le solvant. On obtient 0,57 g du produit attendu sous forme de solide blanc, F = 114°C.
B) Acide 5-(4-chlorophényl)-1-(2,4-dichlorophényl)-4-fluorométhylpyrazole-3-carboxylique.
   A une solution de 0,49 g du composé obtenu à l'étape précédente dans 50 ml de MeOH, on ajoute à TA 10 ml d'une solution à 30 % de NaOH et laisse 10 minutes sous agitation à TA. On verse le mélange réactionnel dans 60 ml d'une solution à 2,5 % d'H₂SO₄, extrait au DCM, lave la phase organique par une solution saturée de NaCl, sèche sur MgSO₄ et évapore sous vide le solvant. On obtient 0,45 g du produit attendu sous forme de solide blanc, F = 60-80°C.

### EXEMPLE 1

### N-(pipéridin-1-yl)-5-(4-chlorophényl)-1-(2,4-dichlorophényl)-4-hydroxyméthylpyrazole-3-carboxamide.

On place 1,75 g du composé de la PREPARATION 2 dans 30 ml de DCM et on ajoute successivement 1,53 ml de triéthylamine, 0,50 ml de 1-aminopipéridine puis, à 0°C, 2,33 g de BOP. On laisse sous agitation à 0°C pendant 10 minutes puis à TA pendant 3 heures et demie. On verse le milieu réactionnel dans 100 ml d'eau glacée, extrait au DCM, lave à l'eau, par une solution saturée de NaCl, puis sèche sur MgSO₄. Le résidu obtenu est chromatographié sur silice fine en éluant par AcOEt/toluène (20/80 à 40/60 ; v/v). On obtient 800 mg du composé attendu, F = 118°C.
RMN
1,1 à 1,7 ppm : m : 6H
2,7 ppm : t : 4H
4,4 ppm : d : 2H
5,15 ppm : t : 1H
7,1 à 7,45 ppm : système AA'-BB' : 4H
7,5 ppm : d. de d. : 1H
7,7 ppm : m : 2H
9,35 ppm:s: 1H

### EXEMPLE 2

### N-(pipéridin-1-yl)-5-(4-chlorophényl)-1-(2,4-dichlorophényl)-4-((2-hydroxyéthoxy)méthyl)pyrazole-3-carboxamide

A une solution de 420 mg du composé de la PREPARATION 3 dans 20 ml de DCM, on ajoute successivement 0,33 ml de triéthylamine, 0,11 ml de 1-aminopipéridine, puis, à 0°C, 0,50 g de BOP. On laisse sous agitation 10 minutes à 0°C puis 3 heures à TA. On reprend par 120 ml d'eau glacée, extrait au DCM, lave à l'eau puis par une solution saturée de NaCl. Après séchage sur MgSO₄, le résidu est chromatographié sur silice fine en éluant par AcOEt/toluène (20/80 à 40/60 ; v/v). On obtient 210 mg du composé attendu, F = 194°C.
RMN
1,2 à 1,7 ppm : m : 6H
2,75 ppm : t : 4H
3,45 ppm : m : 4H
4,4 à 4,7 ppm : m : 3H
7,2 à 7,5 ppm : système AA'-BB' : 4H
7,6 ppm : d. de d. : 1H
7,7 à 7,8 ppm : m : 2H
9,35 ppm : s : 1H

### EXEMPLE 3

### N-(pipéridin-1-yl)-5-(4-chlorophényl)-1-(2,4-dichlorophényl)-4-fluorométhylpyrazole-3-carboxamide.

Une solution de 600 mg du composé de l'EXEMPLE 1 dans 24 ml de DCM est ajoutée goutte à goutte à une solution de 0,19 ml de trifluorure de diéthylaminosulfure dans 24 ml de DCM à -78°C, sous azote. On laisse revenir lentement à 0°C puis agite le mélange une heure à 0°C. Le mélange réactionnel est versé dans une solution saturée de NaHCO₃, puis on extrait au DCM, lave par une solution saturée de NaCl et sèche sur MgSO₄. Le résidu est chromatographié sur silice fine en éluant par AcOEt/toluène (5/95 à 40/60 ; v/v). On obtient 250 mg du produit attendu, F = 184°C.
RMN
1,3 à 1,8 ppm : m : 6H
2,85 ppm : t : 4H
5,6 ppm : d : 2H
7,3 à 7,6 ppm : système AA'-BB' : 4H
7,7 ppm : d. de d. : 1H
7,85 ppm d : 1H
7,95 ppm : d : 1H
9,25 ppm : s : 1H

On peut également préparer ce composé en suivant le mode opératoire décrit ci-dessous.

A une solution de 0,065 g du composé obtenu à la PREPARATION 10 dans 3 ml de toluène, on ajoute à TA 0,036 ml de chlorure de thionyle puis place le mélange réactionnel dans un bain d'huile à 70°C et chauffe progressivement à 120°C. Après 5 minutes, on fait barbotter de l'azote dans le mélange réactionnel par intermittence et poursuit le chauffage pendant 10 minutes. On refroidit le mélange réactionnel à TA et concentre sous vide. On dissout le résidu dans 5 ml de DCM et ajoute cette solution à une solution de 0,021 ml de 1-aminopipéridine et 0,054 ml de triéthylamine dans 5 ml de DCM refroidie à 0°C. Après 30 minutes sous agitation à TA, on verse le mélange réactionnel dans une solution à 2,5 % d'H₂SO₄, extrait au DCM, lave la phase organique par une solution saturée de NaCl, sèche sur MgSO₄ et évapore sous vide le solvant. On obtient 0,07 g du produit attendu.

### EXEMPLE 4

### N-(pipéridin-1-yl)-5-(4-chlorophényl)-1-(2,4-dichlorophényl)-4-(méthylthiométhyl)pyrazole-3-carboxamide.

On place 1 g du composé de la PREPARATION 4 bis dans 15 ml de DCM et l'on ajoute goutte à goutte une solution de 0,23 ml de 1-aminopipéridine et 0,29 ml de triéthylamine dans 15 ml de DCM. Après 16 heures sous agitation à TA, on verse dans 100 ml d'eau glacée. On extrait au DCM, lave à l'eau puis par une solution saturée de NaCl. Le produit obtenu cristallise dans le mélange (iPr)₂O/DCM. On obtient 0,67 g du produit attendu, F = 183°C.
RMN
1,1 à 1,7 ppm : m : 6H
1,8 ppm : s : 3H
2,7 ppm : t : 4H
3,8 ppm : s : 2H
7,1 à 7,4 ppm : système AA'-BB' : 4H
7,5 ppm : d : 1H
7,65 ppm : s : 1H
7,7 ppm : d : 1H
9,15 ppm : s : 1H

### EXEMPLE 5

### Chlorhydrate de N-(pipéridin-1-yl)-5-(4-chlorophényl)-1-(2,4-dichlorophényl)-4-méthoxyméthylpyrazole-3-carboxamide.

On place le produit obtenu à la PREPARATION 5 bis dans 10 ml de DCM et on l'ajoute goutte à goutte à une solution refroidie à 0°C de 0,24 ml de 1-aminopipéridine et 0,33 ml de triéthylamine dans 10 ml de DCM. On laisse 16 heures sous agitation à TA puis on extrait au DCM et lave à l'eau puis par une solution saturée de NaCl. Pour préparer le sel, le produit obtenu est dissous dans l'éther et additionné d'éther chlorhydrique. Le solide obtenu est filtré puis recristallisé dans le mélange MeOH/Et₂O. On obtient 0,16 g du produit attendu, F = 198°C.
RMN
1,3 à 1,9 ppm : s : 6H
3,25 ppm : t : 4H
3,2 ppm : s : 3H
4,45 ppm : s : 2H
5,9 ppm : s.e. : 1H
7,2 à 7,5 ppm système AA'-BB' : 4H
7,6 ppm : d. de d. : 1H
7,7 à 7,9 ppm : m : 2H
10,85 ppm : s.e. : 1H

### EXEMPLE 6

### N-(pipéridin-1-yl)-5-(4-chlorophényl)-1-(2,4-dichlorophényl)-4-cyanométhylpyrazole-3-carboxamide.

On laisse 3 heures sous agitation à TA un mélange de 1,1 g du composé obtenu à la PREPARATION 7 bis, 0,3 ml de 1-aminopipéridine et 0,39 ml de triéthylamine dans 15 ml de DCM. On concentre sous vide le solvant. On chromatographie le résidu sur silice, en éluant par le mélange toluène/AcOEt (95/5 ; v/v) puis (90/10 ; v/v). On obtient 0,066 g du produit attendu après cristallisation dans l'éther iso, F = 123°C.
RMN
1,2 à 1,8 ppm : m : 6H
2,8 ppm : t : 4H
4,05 ppm : s : 2H
7,2 à 7,5 ppm : système AA'-BB' : 4H
7,6ppm : d de d : 1H
7,8 ppm : d : 1H
7,85 ppm : d : 1H
9,45 ppm : s : 1H

### EXEMPLE 7

### N-(pipéridin-1-yl)-5-(4-chlorophényl)-1-(2,4-dichlorophényl)-4-(méthylsulfonylméthyl)pyrazole-3-carboxamide.

On refroidit à 0°C une solution de 0,14 g de 1-aminopipéridine et 1,3 ml de triéthylamine dans 10 ml de DCM, ajoute goutte à goutte une solution de 0,53 g du composé obtenu à la PREPARATION 9 bis et laisse 3 heures sous agitation à TA. On lave le mélange réactionnel à l'eau, par une solution saturée de NaCl, sèche la phase organique sur MgSO₄ et évapore sous vide le solvant. On chromatographie le résidu sur silice en éluant par le mélange toluène/AcOEt (70/30 ; v/v). On obtient 0,38 g du produit attendu, F = 171°C.
RMN
1,2 à 1,8 ppm : m : 6H
2,85 ppm : t : 4H
2,9 ppm : s : 3H
4,7 ppm : s : 2H
7,4 à 7,6 ppm : système AA'-BB' : 4H
7,65 ppm : d de d : 1H
7,85 ppm : d : 1H
7,9 ppm : d : 1H
9,45 ppm : s : 1H

## Revendications

1. Composé de formule : 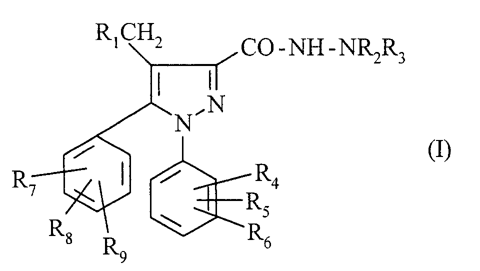 dans laquelle :
- R₁ représente un fluor, un hydroxy, un (C₁-C₅)alcoxy, un (C₁-C₅)alkylthio, un hydroxy(C₁-C₅)alcoxy, un groupe -NR₁₀R₁₁, un cyano, un (C₁-C₅)alkylksulfonyle, un (C₁-C₅)alkylsulfinyle ;
- R₂ et R₃ représentent un (C₁-C₄)alkyle ou ensemble avec l'atome d'azote auquel ils sont liés constituent un radical hétérocyclique, saturé ou insaturé, de 5 à 10 chaînons, non substitué ou substitué une ou plusieurs fois par un (C₁-C₃)alkyle ou par un (C₁-C₃)alcoxy;
- R₄, R₅, R₆, R₇, R₈, R₉ représentent chacun indépendamment l'hydrogène, un halogène, un trifluorométhyle, et lorsque R₁ représente un fluor, R₄, R₅, R₆, R₇, R₈ et/ou R₉ peuvent également représenter un fluorométhyle ; et à la condition que l'un au moins des substituants R₄ ou R₇ soit différent de l'hydrogène ;
- R₁₀ et R₁₁ représentent chacun indépendamment l'hydrogène ou un (C₁-C₅)alkyle ou R₁₀ et R₁₁ ensemble avec l'atome d'azote auquel ils sont liés constituent un radical hétérocyclique choisi parmi pyrrolidin-1-yle, pipéridin-1-yle, morpholin-4-yle, pipérazin-1-yle non substitué ou substitué par un (C₁-C₄)alkyle ; ainsi que ses sels et leurs solvates.

2. Composé selon la revendication 1 de formule : 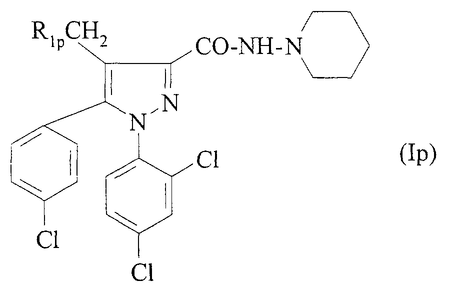 dans laquelle R₁ₚ représente un fluor, un méthoxy ou un méthylthio, ainsi que ses sels et leurs solvates.

3. Composé selon la revendication 1 ou 2, qui est sous forme radiomarquée.

4. Composé selon la revendication 3, de formule (I) ou (Ip), dans laquelle R₁ ou, respectivement, R₁ₚ, est ¹⁸F.

5. Procédé pour la préparation d'un composé de formule (I) selon la revendication 1, de ses sels et de leurs solvates, caractérisé en ce que :
a) on traite un ester bromé de formule : 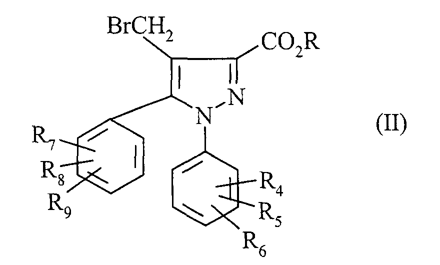 dans laquelle R₄, R₅, R₆, R₇, R₈, R₉ sont tels que définis pour (I) dans la revendication 1 et R représente un (C₁-C₄)alkyle, par un composé de formule : R'₁ A (III) dans laquelle R'₁ représente R₁ tel que défini pour (I) dans la revendication 1 ou un précurseur de R₁ et A représente un hydrogène ou un cation ;
b₁) dans l'ester ainsi obtenu, on transforme éventuellement R'₁ en R₁ ;
b₂) on saponifie l'ester obtenu à l'étape a) ou à l'étape b₁), de formule : 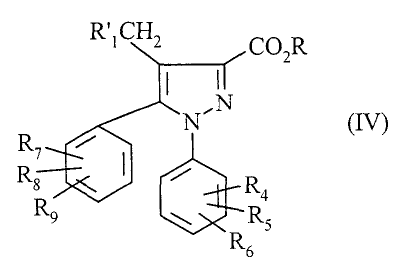
c) on traite l'acide ainsi obtenu de formule : 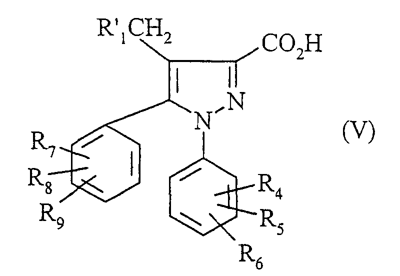 ou un dérivé fonctionnel de cet acide, avec une hydrazine de formule : H₂N-NR₂R₃ (VI) dans laquelle R₂ et R₃ sont tels que définis pour (I) dans la revendication 1 ;
d₁) dans le composé ainsi obtenu, on transforme éventuellement R'₁ en R₁ ; et
d₂) éventuellement, on transforme le composé obtenu à l'étape c) ou l'étape d₁) de formule : 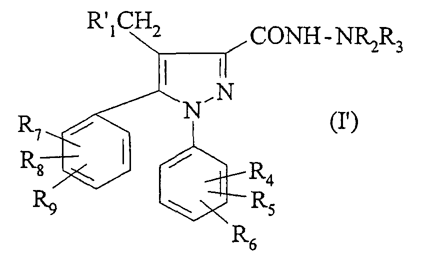 en l'un de ses sels ou de leurs solvates.

6. Procédé pour la préparation d'un composé de formule (I) selon la revendication 1 dans laquelle R₁ = R₁ₐ et représente un groupe choisi parmi un fluor, un (C₁-C₅)alcoxy, un (C₁-C₅)alkylthio, un hydroxy(C₁-C₅)alcoxy, un cyano ou un groupe -NR₁₀R₁₁ dans lequel R₁₀ et R₁₁ sont tels que définis pour (I) dans la revendication 1, caractérisé en ce que :
a) on traite un ester bromé de formule : 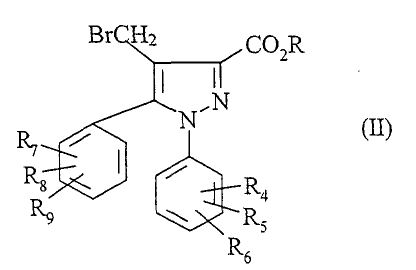 dans laquelle R₄, R₅, R₆, R₇, R₈, R₉ et R sont tels que définis dans la revendication 5 par un hydroxyde de métal alcalin ou alcalino-terreux ;
b) on traite l'acide ainsi obtenu de formule : 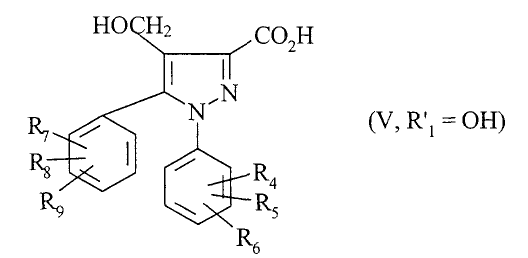 ou un dérivé fonctionnel de cet acide, avec une hydrazine de formule H₂N-NR₂R₃ (VI) dans laquelle R₂ et R₃ sont tels que définis pour (I) dans la revendication 1 ;
c) on traite le composé ainsi obtenu de formule : 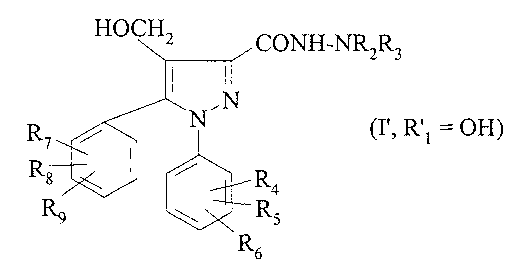 par un composé de formule Hal-B dans laquelle Hal représente un halogène et B représente un radical mésyle, tosyle ou trifluorométhanesulfonyle ;
d) on traite le composé ainsi obtenu de formule : 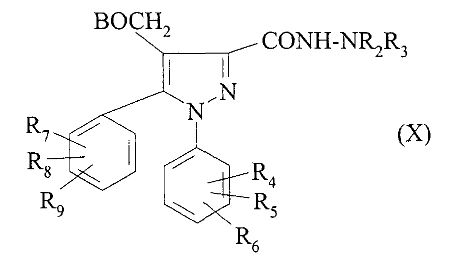 par un composé de formule R₁ₐ A (XI) dans laquelle R₁ₐ est tel que défini ci-dessus et A représente un hydrogène ou un cation ; et
e) éventuellement, on transforme le composé ainsi obtenu de formule : 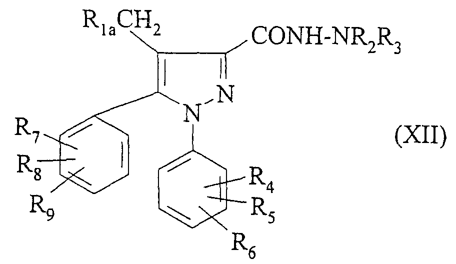 en l'un de ses sels ou de leurs solvates.

7. Composition pharmaceutique, caractérisée en ce qu'elle comprend à titre de principe actif un composé selon la revendication 1 ou 2.

8. Composition pharmaceutique selon la revendication 7, sous forme d'unité de dosage, dans laquelle le principe actif est mélangé à au moins un excipient pharmaceutique.

9. Composition pharmaceutique selon la revendication 8, contenant 0,5 à 1000 mg de principe actif.

10. Composition pharmaceutique selon la revendication 9, contenant 2 à 200 mg de principe actif.

11. Utilisation d'un composé selon la revendication 1 de formule (I), dans laquelle R₁ est un hydroxy ou hydroxy(C₁-C₅)alcoxy, pour la préparation de ligands permettant d'isoler, purifier et caractériser les récepteurs aux cannabinoïdes.

## Patentansprüche

1. Verbindung der Formel 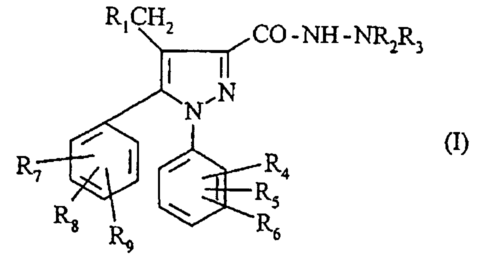 worin
- R₁ Fluor, Hydroxy, (C₁-C₅)-Alkoxy, (C₁-C₅)-Alkylthio, Hydroxy-(C₁-C₅)-alkoxy, eine Gruppe -NR₁₀R₁₁, Cyano, (C₁-C₅)-Alkylsulfonyl, (C₁-C₅)-Alkylsulfinyl darstellt;
- R₂ und R₃ (C₁-C₄)-Alkyl darstellen oder zusammen mit dem Stickstoffatom, an das sie gebunden sind, einen 5- bis 10-gliedrigen gesättigten oder ungesättigen heterocyclischen Rest bilden, der gegebenenfalls ein- oder mehrmals durch (C₁-C₃)-Alkyl oder (C₁-C₃)-Alkoxy substituiert ist;
- R₄, R₅, R₆, R₇, R₈, R₉ jeweils unabhängig voneinander Wasserstoff, ein Halogen oder Trifluormethyl darstellen und, wenn R₁ Fluor darstellt, R₄, R₅, R₆, R₇, R₈ und/oder R₉ auch Fluormethyl darstellen können; und mit der Maßgabe, dass mindestens einer der Substituenten R₄ oder R₇ nicht Wasserstoff ist;
- R₁₀ und R₁₁ jeweils unabhängig voneinander Wasserstoff oder (C₁-C₅)-Alkyl darstellen oder R₁₀ und R₁₁ zusammen mit dem Stickstoffatom, an das sie gebunden sind, einen heterocyclischen Rest ausgewählt aus Pyrrolidin-1-yl, Piperidin-1-yl, Morpholin-4-yl, Piperazin-1-yl gegebenenfalls durch (C₁-C₄)-Alkyl substituiert, bilden;
sowie ihre Salze und die Solvate derselben.

2. Verbindung nach Anspruch 1 der Formel 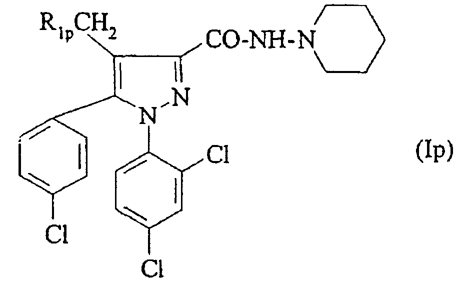 worin R₁ₚ Fluor, Methoxy oder Methylthio darstellt, sowie ihre Salze und die Solvate derselben.

3. Verbindung nach Anspruch 1 oder 2, welche in radiomarkierter Form vorliegt.

4. Verbindung nach Anspruch 3 der Formel (I) oder (Ip), worin R₁ bzw. R₁ₚ für ¹⁸F steht.

5. Verfahren zur Herstellung einer Verbindung der Formel (I) nach Anspruch 1, ihrer Salze und der Solvate derselben, dadurch gekennzeichnet, dass
a) ein Bromester der Formel 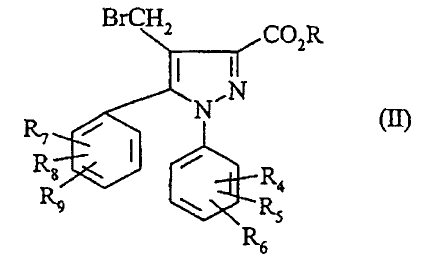 worin R₄, R₅, R₆, R₇, R₈, R₉ wie in Anspruch 1 für (I) definiert sind und R (C₁-C₄)-Alkyl darstellt, mit einer Verbindung der Formel: R'₁ A (III), worin R'₁ R₁ wie in Anspruch 1 für (I) definiert oder einen Vorläufer von R₁ darstellt und A Wasserstoff oder ein Kation darstellt;
b₁) in dem so erhaltenen Ester R'₁ gegebenenfalls in R₁ übergeführt wird;
b₂) der in Schritt a) oder in Schritt b₁) erhaltene Ester der Formel: 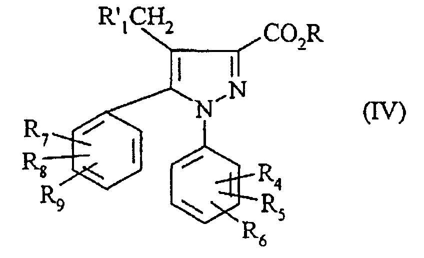 verseift wird;
c) die so erhaltene Säure der Formel: 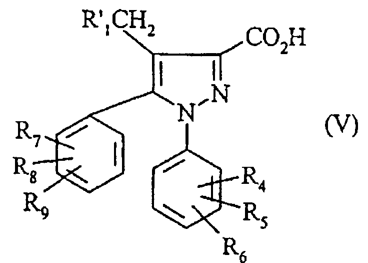 oder ein funktionelles Derivat dieser Säure mit einem Hydrazin der Formel: H₂N-NR₂R₃ (VI), worin R₂ und R₃ wie in Anspruch 1 für (I) definiert sind, behandelt wird;
d₁) in der so erhaltenen Verbindung R'₁ gegebenenfalls in R₁ übergeführt wird; und
b₂) die in Schritt c) oder in Schritt d₁) erhaltene Verbindung der Formel: 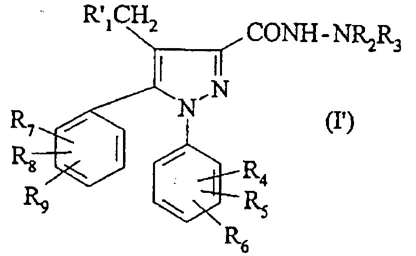 gegebenenfalls in eines ihrer Salze oder eines der Solvate derselben übergeführt wird.

6. Verfahren zur Herstellung einer Verbindung der Formel (I) nach Anspruch 1, worin R₁ = R₁ₐ und eine Gruppe ausgewählt aus Fluor, (C₁-C₅)-Alkoxy, (C₁-C₅)-Alkylthio, Hydroxy-(C₁-C₅)-alkoxy, Cyano oder einer Gruppe -NR₁₀R₁₁ darstellt, worin R₁₀ und R₁₁ wie in Anspruch 1 für (I) definiert sind, dadurch gekennzeichnet, dass
a) ein Bromester der Formel: 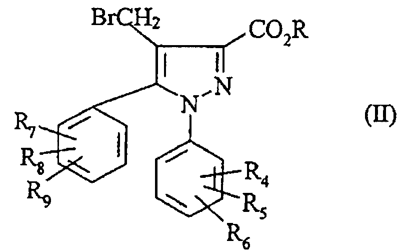 worin R₄, R₅, R₆, R₇, R₈, R₉ und R wie in Anspruch 5 definiert sind, mit einem Alkalimetall- oder einem Erdalkalimetallhydroxid behandelt wird;
b) die so erhaltene Säure der Formel: 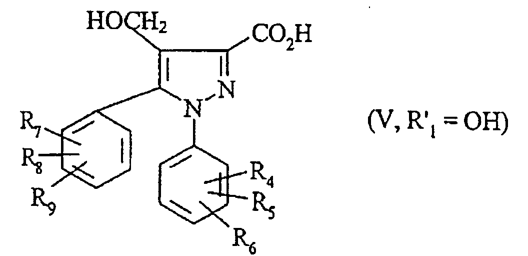 oder ein funktionelles Derivat dieser Säure mit einem Hydrazin der Formel: H₂N-NR₂R₃ (VI), worin R₂ und R₃ wie in Anspruch 1 für (I) definiert sind, behandelt wird;
c) die so erhaltene Verbindung der Formel: 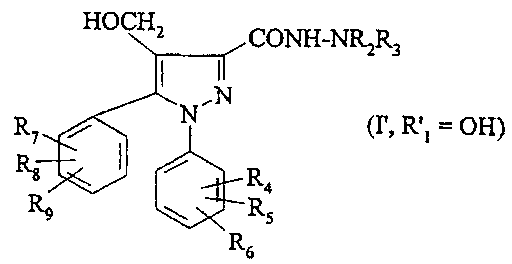 mit einer Verbindung der Formel Hal-B behandelt wird, in welcher Hal ein Halogen und B einen Mesyl-, Tosyl- oder Trifluormethansulfonylrest darstellt;
d) die so erhaltene Verbindung der Formel: 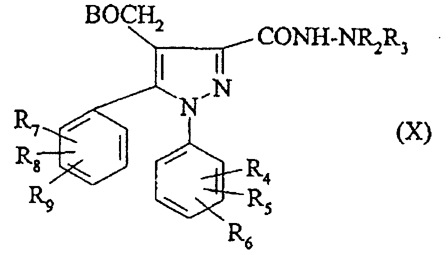 mit einer Verbindung der Formel R₁ₐ A (XI) behandelt wird, worin R₁ₐ wie oben definiert ist und A Wasserstoff oder ein Kation darstellt; und
e) die so erhaltene Verbindung der Formel: 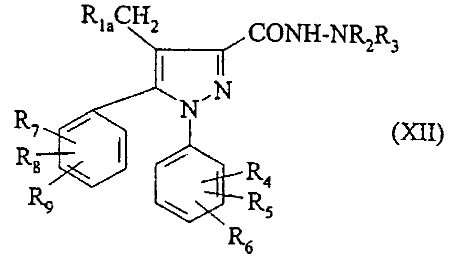 gegebenenfalls in eines ihrer Salze oder eines der Solvate derselben übergeführt wird.

7. Pharmazeutische Zusammensetzung, dadurch gekennzeichnet, dass sie als Wirkstoff eine Verbindung nach Anspruch 1 oder 2 enthält.

8. Pharmazeutische Zusammensetzung nach Anspruch 7 in Dosiseinheitsform, worin der Wirkstoff mit mindestens einem pharmazeutischen Exzipienten vermischt ist.

9. Pharmazeutische Zusammensetzung nach Anspruch 8, die 0,5 bis 1000 mg Wirkstoff enthält.

10. Pharmazeutische Zusammensetzung nach Anspruch 9, die 2 bis 200 mg Wirkstoff enthält.

11. Verwendung einer Verbindung nach Anspruch 1 der Formel (I), worin R₁ Hydroxy oder Hydroxy-(C₁-C₅)-alkoxy ist, zur Herstellung von Liganden, die eine Isolierung, Reinigung und Charakterisierung von Cannabinoid-Rezeptoren gestatten.

## Claims

1. A compound of the formula 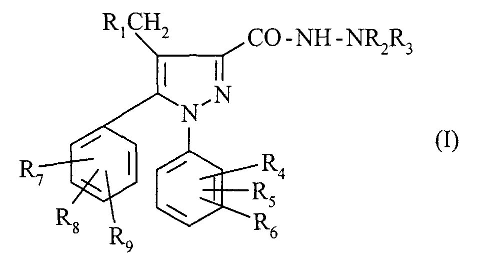 in which:
- R₁ is a fluorine, a hydroxyl, a (C₁-C₅)alkoxy, a (C₁-C₅)alkylthio, a hydroxy(C₁-C₅)alkoxy, a group -NR₁₀R₁₁, a cyano, a (C₁-C₅)alkylsulfonyl or a (C₁-C₅)-alkylsulfinyl;
- R₂ and R₃ are a (C₁-C₄)alkyl or, together with the nitrogen atom to which they are bonded, form a 5- to 10-membered, saturated or unsaturated heterocyclic radical which is unsubstituted or monosubstituted or polysubstituted by a (C₁-C₃)alkyl or by a (C₁-C₃)alkoxy;
- R₄, R₅, R₆, R₇, R₈ and R₉ are each independently hydrogen, a halogen or a trifluoromethyl, and if R₁ is a fluorine, R₄, R₅, R₆, R₇, R₈ and/or R₉ can also be a fluoromethyl, with the proviso that at least one of the substituents R₄ or R₇ is other than hydrogen; and
- R₁₀ and R₁₁ are each independently hydrogen or a (C₁-C₅)alkyl, or R₁₀ and R₁₁, together with the nitrogen atom to which they are bonded, form a heterocyclic radical selected from pyrrolidin-1-yl, piperidin-1-yl, morpholin-4-yl and piperazin-1-yl, which is unsubstituted or substituted by a (C₁-C₄)alkyl;
its salts and their solvates.

2. The compound according to claim 1 of the formula 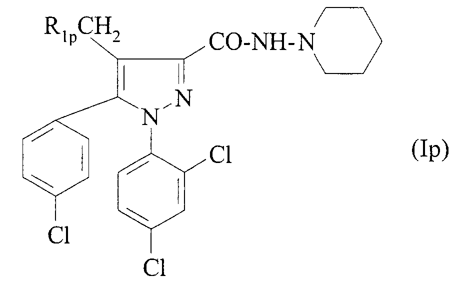 in which R₁ₚ is a fluorine, a methoxy or a methylthio, its salts and their solvates.

3. The compound according to claim 1 or 2 which is in radiolabelled form.

4. The compound according to claim 3 of formula (I) or (Ip), in which R₁ or R₁ₚ, respectively, is ¹⁸F.

5. A method of preparing a compound of formula (I) according to claim 1, its salts and their solvates, characterized in that:
a) a brominated ester of the formula 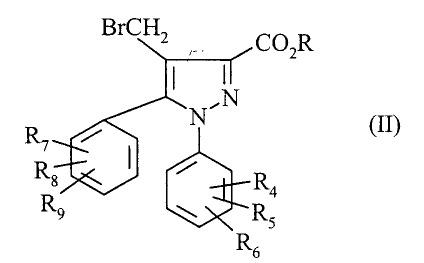 in which R₄, R₅, R₆, R₇, R₈ and R₉ are as defined for (I) in claim 1 and R is a (C₁-C₄)alkyl, is treated with a compound of the formula R'₁A (III), in which R'₁ is R₁ as defined for (I) in claim 1, or a precursor of R₁, and A is a hydrogen or a cation;
b1) in the resulting ester, R'₁ is optionally converted to R₁;
b2) the ester obtained in step a) or step bl), of the formula 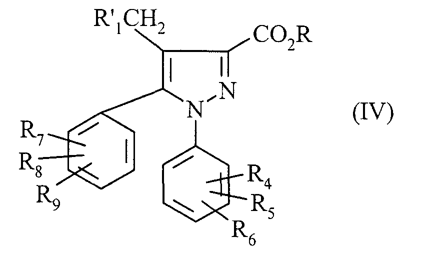 is saponified;
c) the resulting acid of the formula 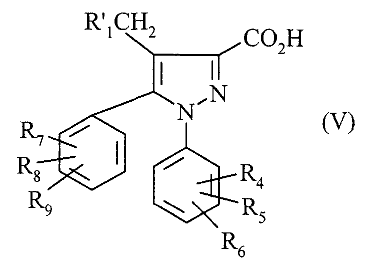 or a functional derivative of this acid, is treated with a hydrazine of the formula H₂N-NR₂R₃ (VI), in which R₂ and R₃ are as defined for (I) in claim 1 ;
d1) in the resulting compound, R'₁ is optionally converted to R₁; and
d2) the compound obtained in step c) or step dl), of the formula 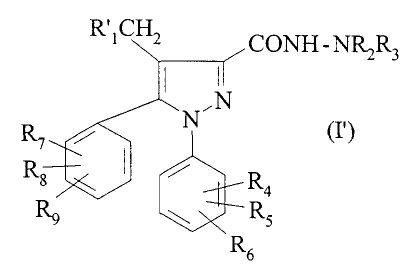 is optionally converted to one of its salts or one of their solvates.

6. A method of preparing a compound of formula (I) according to claim 1 in which R₁ = R₁ₐ and is a group selected from a fluorine, a (C₁-C₅)alkoxy, a (C₁-C₅)alkylthio, a hydroxy(C₁-C₅)alkoxy, a cyano and a group -NR₁₀R₁₁, in which R₁₀ and R₁₁ are as defined for (I) in claim 1, characterized in that:
a) a brominated ester of the formula 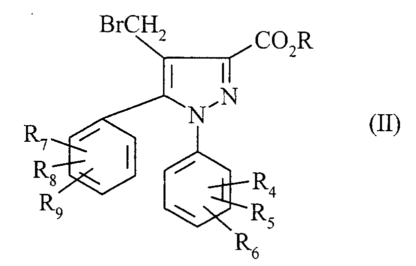 in which R₄, R₅, R₆, R₇, R₈, R₉ and R are as defined in claim 5, is treated with an alkali metal or alkaline earth metal hydroxide;
b) the resulting acid of the formula 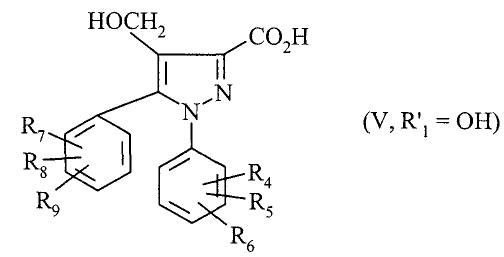 or a functional derivative of this acid, is treated with a hydrazine of the formula H₂N-NR₂R₃ (VI), in which R₂ and R₃ are as defined for (I) in claim 1;
c) the resulting compound of the formula 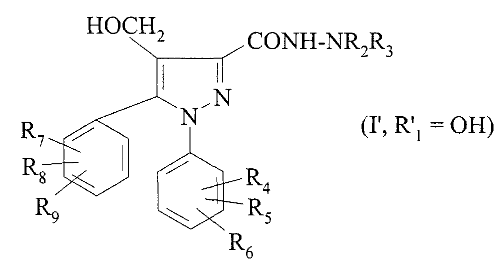 is treated with a compound of the formula Hal-B, in which Hal is a halogen and B is a mesyl, tosyl or trifluoromethanesulfonyl radical;
d) the resulting compound of the formula 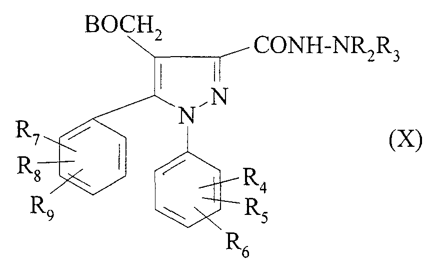 is treated with a compound of the formula R₁ₐA (XI), in which R₁ₐ is as defined above and A is a hydrogen or a cation; and
e) the resulting compound of the formula 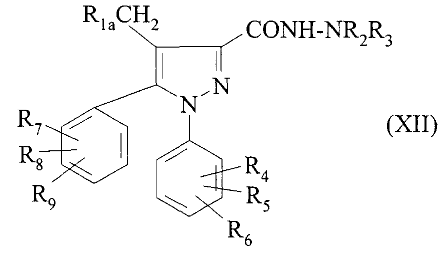 is optionally converted to one of its salts or one of their solvates.

7. A pharmaceutical composition, characterized in that it comprises, as the active principle, a compound according to claim 1 or 2.

8. The pharmaceutical composition according to claim 7 in the form of a dosage unit, in which the active principle is mixed with at least one pharmaceutical excipient.

9. The pharmaceutical composition according to claim 8 containing 0.5 to 1000 mg of active principle.

10. The pharmaceutical composition according to claim 9 containing 2 to 200 mg of active principle.

11. Use of a compound according to claim 1 of formula (I) in which R₁ is a hydroxyl or hydroxy(C₁-C₅)alkoxy for the preparation of ligands which make it possible to isolate, purify and characterize the cannabinoid receptors.
